Europäisches Patentamt

European Patent Office

Office européen des brevets

(11) Publication number : **0 507 587 A2**

## (12) EUROPEAN PATENT APPLICATION

(21) Application number : **92302913.6**

(22) Date of filing : **02.04.92**

(51) Int. Cl.⁵ : **G01N 33/68,** G01N 33/576,
// G01N33/543

(30) Priority : **03.04.91 US 679693**

(43) Date of publication of application :
**07.10.92 Bulletin 92/41**

(84) Designated Contracting States :
**AT BE CH DE DK ES FR GB GR IT LI LU NL PT
SE**

(71) Applicant : **SYNTEX (U.S.A.) INC.
3401 Hillview Avenue
Palo Alto California 94304 (US)**

(72) Inventor : **Rejman, John J.
13619 Highway 70 East
Lenior City TN 37771 (US)**
Inventor : **Weng, Litai
995 N. California Avenue
Palo Alto CA 94303 (US)**
Inventor : **Varro, Rudolph
3402 Woodstock Lane
Mt. View CA 94040 (US)**

(74) Representative : **Nicholls, Kathryn Margaret et
al
Mewburn Ellis, 2 Cursitor Street
London EC4A 1BQ (GB)**

(54) **Immunoassay for immunoglubulins.**

(57)    A method for carrying out an immunoassay for an immunoglobulin in which a sample suspected of containing the immunoglobulin and reagents useful for detecting the immunoglobulin of interest are combined in a single step in an aqueous medium, wherein one of the reagents includes a small molecule bound to a receptor for the immunoglobulin, one includes an antigen capable of binding to the immunoglobulin and one includes a signal generating means bound to a receptor for the antigen capable of binding to a site on the antigen different from the site of binding of the immunoglobulin.

EP 0 507 587 A2

Jouve, 18, rue Saint-Denis, 75001 PARIS

Immunoglobulins are a class of proteins which function as antibodies and are produced by specialized cells (β-lymphocytes) of an immuno-competent organism (i.e., an organism with an intact immune system) in response to the presence of a molecule which is foreign to the organism.

There are five categories of immunoglobulins (Ig) based upon their chemical structures and the different functions they perform in the immune system: IgA, IgD, IgE, IgG and IgM. From a diagnostic viewpoint, the IgM immunoglobulin is of great interest since it is the first to appear in serum following the body's exposure to an antigenic substance.

Accordingly, immunoassays to identify the presence of immunoglobulins and in particular IgM, are useful for the early diagnosis of infection. Hepatitis is an infectious disease for which an immunoassay based upon immunoglobulin detection is useful. Such an assay finds usefulness not only as a purely diagnostic tool, but also in pre-transfusion screening of blood and in screening potential blood donors. Hepatitis A or infectious hepatitis occurs worldwide, sporadically and epidemically, with a tendency towards cyclic recurrences. Presence of the hepatitis A virus (HAV) is typically established by verifying the presence of IgM antibodies in the serum of acutely or recently ill patients by a radioimmunoassay or enzyme immunoassay. Hepatitis B or serum hepatitis also occurs worldwide and is commonly seen among drug users who transmit the disease via shared hypodermic needles. Hepatitis B is usually diagnosed by confirmation of the presence of viral antigens. Assay kits for the hepatitis B surface antigen, the core antigen and antibodies directed against these antigens are commercially available.

U. S. Patent No. 4,292,403 pertains to an Ig immunoassay where bound anti-Ig is incubated with a sample, then incubated with an antigen capable of binding to Ig, and lastly incubated with a labeled anti-antigen fragment. Each incubation is followed by a wash step and the antibody is bound by coupling to a solid carrier by means of covalent bonds or by adsorption.

U. S. Patent No. 4,273,756 describes an Ig assay where bound anti-Ig is contacted with a test sample, washed, then reacted with a labeled antigen capable of binding to Ig.

U. S. Patent No. 4,020,151 pertains to an Ig assay where Ig in a sample is first sorbed directly onto a nonimmunological surface, followed by reaction with labeled anti-Ig.

U. S. Patent No. 4,837,167 describes a simultaneous sandwich assay to determine multi-determinant antigens using high affinity monoclonal anti-Ig.

Bradley, et al., Journal of Clinical Microbiology 5(5):521-530 (1977) describes an assay for IgM class immunoglobulins related to the HAV virus where HAV and radiolabeled IgG anti-HAV is added to a test tube coated with patient serum suspected of containing IgM.

U. S. Patent No. 4,474,878 pertains to a sandwich enzyme immunoassay for hepatitis antigen where a sample is incubated with bound antibody, then incubated with enzyme labeled antibody.

U. S. Patent No. 4,818,688 describes an assay for antibodies to hepatitis B core antigen (HBcAg) where labeled antibody competes with antibody in the sample for binding to immobilized HBcAg.

U. S. Patent No. 4,098,876 pertains to a reverse sandwich assay for hepatitis associated antigen where a sample is incubated with labeled antibody followed by incubation with immobilized antibody.

Bussian, et al., Clinical Chemistry 34(6):1315 (1988) describes an assay for IgM antibodies to HAV: sample is incubated with biotinylated anti-IgM then with a streptavidin coated solid phase; the complex is washed then mixed with HAV; and the new complex is washed and then mixed with labeled anti-HAV.

U. S. Patent No. 4,271,140 pertains to the use of biotin and avidin in a double receptor complex where biotin and avidin form a nonimmune, reversible binding system.

U. S. Patent No. 4,298,685 describes the use of a biotinylated antibody bound to an avidin coated solid phase, where analyte present in a sample competes with unlabeled analyte for binding to the antibody.

U. S. Patent No. 4,935,339 pertains to a one step immunoassay where a biotinylated first immunological binding partner, a test sample, a labeled second immunological binding partner and a biotin-binding protein bound to a carrier are all combined.

U. S. Patent No. 4,343,896 describes an assay using two antibodies from different animal species which are raised against the same antigen, where one antibody is labeled and the other antibody is insolubilized by a third antibody.

U. S. Patent Application Serial No. 07/389,659 filed February 4, 1991 (corresponding to European Patent Publication No. 411,945, Japanese Patent Application No. 206628/90 and Canadian Patent Application No. SN 2,022,517-3) pertains to an immunoassay using a small molecule bound to a specific binding pair member and a second small molecule bound to a second specific binding pair member, where both binding pair members are capable of binding the analyte. The assay also uses a receptor for the first small molecule, bound to a support and a receptor for the second small molecule bound to a label.

According to one aspect of the present invention a method for carrying out a qualitative immunoassay for specific immunoglobulins is provided wherein the following components are combined without prior incubation in an aqueous medium, and incubated: a sample suspected of containing the immunoglobulin of interest, a small molecule bound to a receptor for the immunoglobulin, an antigen capable of binding to the immunoglobulin, a signal generating means bound to a receptor for the antigen where the receptor is capable of binding to a site on the antigen different from the site of binding of the immunoglobulin, and a support to which is bound a receptor for the small molecule. The medium is then separated from the support. The medium or the support is observed for the presence or amount of the signal generating means, the presence or amount thereof being related to the presence or amount of immunoglobulin in the sample.

Another embodiment of the invention describes a method for carrying out an immunoassay for an IgM-type immunoglobulin wherein the following components are combined without prior incubation in an aqueous medium, and incubated: a sample suspected of containing IgM, a biotinylated antibody capable of binding IgM, an antigen:antibody complex wherein the antigen is capable of binding co IgM and the antibody is bound to a signal generating means, and a receptor for biotin bound to a support. The medium is then separated from the support. The medium or the support is observed for the presence or amount of the signal generating means, the presence or amount thereof being related to the presence or amount of IgM in the sample.

Yet another embodiment of the invention describes an assay for detecting an IgM-type immunoglobulin suspected of being present in a sample wherein a complex is formed in direct relation to the amount of IgM in the sample, the complex being:

$$\text{Label-Ab}_{Ag}: \text{Ag}: \text{IgM}: \text{Ab}_{IgM}\text{-X}:\text{Y-support}$$

where Label-Ab$_{Ag}$ is an antibody for an antigen (Ag) bound to a label, Ag is an antigen capable of being bound to IgM and is selected from the group consisting of hepatitis A virus antigen and hepatitis B core antigen, Ab$_{IgM}$-X is a small molecule (X) bound to an antibody for IgM, and Y-support is a receptor for the small molecule bound to a support.

Another embodiment of the invention describes a composition of matter consisting of a conjugate of a biotinylated antibody bound to an immunoglobulin, an antigen bound to the immunoglobulin and a labeled antibody bound to the antigen.

Still another embodiment of the invention describes a composition of matter:

$$\text{Label-Ab}_{Ag}: \text{Ag}: \text{IgM}: \text{Ab}_{IgM}\text{-X}:\text{Y-support}$$

where Label-Ab$_{Ag}$ is an antibody for an antigen (Ag) bound to a label, IgM is an IgM-type immunoglobulin, Ag is an antigen capable of being bound to IgM and is selected from the group consisting of hepatitis A virus antigen and hepatitis B core antigen, Ab$_{IgM}$-X is a small molecule (X) bound to an antibody for the IgM, and Y-support is a receptor for the small molecule bound to a support.

In another embodiment of the invention a kit for carrying out an immunoassay for an immunoglobulin is described which comprises in packaged form: a conjugate of a small molecule and a receptor for the immunoglobulin, an antigen capable of being bound to the immunoglobulin, a conjugate of a label and a receptor for the antigen, and a support comprised of a surface bound to a receptor for the small molecule.

The present invention generally provides a one step simultaneous immunoassay for specific immunoglobulins (Ig), preferably IgM class and in particular IgM for hepatitis B core antigen and IgM for hepatitis A virus antigen. A complex is formed between the Ig analyte, an antigen:antibody complex and an anti-Ig antibody-small molecule complex. The small molecule can allow for the complex to be insolubilized by means of a receptor for the small molecule bound to a support. All the reagents are mixed together in the assay medium without prior incubation and then incubated. This single incubation is followed by a wash step, which is then followed by the addition of any remaining members of the signal producing system. The antigen:antibody complex may be formed prior to addition to the assay medium, or it may be formed in the assay medium.

There are several advantages to the present invention. One advantage is the elimination of the numerous incubation and wash steps commonly seen in state of the art techniques. Elimination of several of these steps provides for an assay format that is less labor intensive. In some instances, this invention may use a slightly higher amount of reagents, but this is far outweighed by the labor-saving advantage. Another advantage is that by eliminating the need for several incubation and wash steps, this invention provides for shorter assay time. Surprisingly, even with the elimination of the numerous incubation and wash steps, an adequate signal can be obtained to enable detection of the analyte.

Before proceeding further with the description of the specific embodiments of the present invention, a number of terms will be defined.

"Immunoglobulin" means the compound to be measured in a sample that is the material of interest, i.e., the analyte. The sample is preferably serum or plasma. The immunoglobulin (Ig) may be directed against a causative agent of a disease state. The immunoglobulin can be class specific such as IgA, IgD, IgE, IgG and IgM, with a molecular weight that will generally vary from about 160,000 to about $10^6$. As used herein the term "immunoglobulin" shall refer to the analyte and the term "antibody" shall be used in reference to the reagents.

"Antigen" means any compound capable of binding to the immunoglobulin of interest, and against which

EP 0 507 587 A2

antibodies can be raised. For example, the antigen can be hepatitis B core antigen or hepatitis A virus antigen.

"Ligand" means any organic compound for which a receptor naturally exists or can be prepared.

"Receptor" means any compound or composition capable of recognizing a particular spatial and polar organization of a molecule, e.g., epitopic or determinant site. Illustrative receptors include naturally occurring receptors, e.g., thyroxine binding globulin, antibodies, enzymes, Fab fragments, lectins, protein A, complement component C1q, avidin, streptavidin and the like. Preferably, if the small molecule is biotin, the receptor for biotin may be avidin or streptavidin.

"Support" means any non-porous or porous surface. Typical support surfaces include glass or plastic beads, latex or cellulose particles, glass or cellulose filter paper, nitrocellulose membranes, polystyrene plates, magnetic particles, plastic tubes or vessels, and the like. The support may be of any convenient material to which a receptor can be non-diffusively bound and which does not dissolve in or react adversely with the aqueous medium. Usually the support will be plastic such as polystyrene, polycarbonate, polyacrylate, polyurethane, polyvinylchloride, teflon and the like or it may be metallic such as steel, nickel, copper, gold, chromium dioxide and preferably will be ceramic including, for example, quartz, glass, and the like. When the support is a matrix of beads, the beads will usually be of a defined approximately uniform, size, preferably 0.2-2.5mm, and will have either a rough or smooth surface, preferably smooth. Preferably the beads are rounded or oblong, usually approximately spherical and have surface properties which minimize non-specific binding. As used in the immunoassays of the invention, the support will have bound to it a material such as a receptor, preferably such as an antibody, avidin, apoenzyme, repressor protein, intrinsic factor and the like.

Binding of materials to the support or surface may be accomplished by well-known techniques, commonly available in the literature. See, for example, "Immobilized Enzymes," Ichiro Chibata, Halsted Press, New York (1978) and Cuatrecasas, J. Biol. Chem., 245:3059 (1970). The surface can have any one of a number of shapes, such as strip, rod, particle, including bead, and the like.

The surface will usually be polyfunctional or be capable of being polyfunctionalized or be capable of binding a receptor, for example, through specific or non-specific covalent or non-covalent interactions. A wide variety of functional groups are available or can be incorporated. Functional groups include carboxylic acids, aldehydes, amino groups, cyano groups, ethylene groups, hydroxyl groups, mercapto groups and the like. The manner of linking a wide variety of compounds to particles is well known and is amply illustrated in the literature. See for example Cuatrecasas, J. Biol. Chem. 245:3059 (1970). The length of a linking group to the material being linked may vary widely, depending upon the nature of the material being linked, the effect of the distance between the material being linked and the particle on the hybridization of the sequences and the like. The material being linked will be substantially bound to the outer surface of the particle.

Particles employed as the surface can be fluorescent either directly or by virtue of fluorescent compounds or fluorescers bound to the particle in conventional ways. The fluorescers will usually be dissolved in or bound covalently or non-covalently to the particle and will frequently be substantially uniformly bound through the particle. Fluoresceinated latex particles are taught in U.S. Patent No. 3,853,987.

"Small molecule" means an organic or organometallic group, having a molecular weight of from 100-2000, preferably 150-1000, usually bonded to a receptor or a support and for which a receptor exists or can be prepared. Examples of small molecules useful in the invention include derivatives of biotin, lysergic acid, brucine, fluorescein, vitamin $B_{12}$ and in general molecules that are not usually found in high concentration in the samples to be assayed. For biological samples, highly toxic molecules and synthetically derived molecules other than drugs are often preferred.

"Signal generating means" means one or more components, at least one component being a label, which generate a signal that relates to the presence or amount of immunoglobulin in a sample. The signal generating means includes all of the reagents required to produce a measurable signal. The label can be isotopic or non-isotopic, usually non-isotopic, including catalysts such as an enzyme, a chromogen such as a fluorescer, dye or chemiluminescer, a metallic particle, and so forth. The label can be conjugated directly to the receptor for the antigen or the label can be conjugated to a receptor for a small molecule, where the small molecule is bound to the receptor for the antigen. Components of the signal generating means may be chemiluminescers, radioactive substances, coenzymes, substances that react with enzymic products, enzymes, and catalysts, solid particles, fluorophors, chromophors, gold particles and the like. The signal generating means provides a signal detectable by external means, preferably by measurement of the degree of aggregation of particles or by use of electromagnetic radiation, desirably by visual examination. For the most part, the signal generating means will involve, a chromophoric substrate and enzyme, where chromophoric substrates are enzymatically converted to dyes which absorb light in the ultraviolet or visible region, phosphors, fluorescers or chemiluminescers, radioactive atoms, electroactive groups, and the like.

In the methods according to the present invention, the assay medium suspected of containing an immunoglobulin may include a small molecule bound to a receptor for an immunoglobulin, an antigen capable of binding

4

to the immunoglobulin, a signal generating means bound to a receptor for the antigen, said receptor being capable of binding to a site on said antigen different from the site of binding of said immunoglobulin, and a support to which is bound a receptor for said small molecule. The signal generating means is preferably a label selected from the group consisting of enzymes, fluorophors, chemiluminescers and metallic particles, and most preferably said label is an enzyme selected from the group consisting of peroxidase, β-galactosidase, urease, alkaline phosphatase and Q-beta-replicase; the signal generating means may also comprise a second small molecule bound to said receptor for said antigen and a receptor for said small molecule bound to a label selected from the group consisting of enzymes, fluorophors, chemiluminescers and metallic particles, preferably wherein said second small molecule is a fluorescein derivative, said receptor for the small molecule is an antibody, and said label is an enzyme selected from the group consisting of peroxidase, β-galactosidase, urease and alkaline phosphatase.

Also, in an immunoassay methods according to the present invention for an IgM-type immunoglobulin, which includes a biotinylated antibody capable of binding said IgM, an antigen:antibody complex wherein said antigen is capable of binding to said IgM and said antibody is bound to a signal generating means, and a receptor for biotin bound to a support, the signal generating means may be a label selected from the group consisting of enzymes, fluorophors, chemiluminescers and metallic particles, and preferably said label is an enzyme selected from the group consisting of peroxidase, β-galactosidase, urease, alkaline phosphatase and Q-beta-replicase; the signal generating means may comprise a small molecule bound to the antibody and a receptor for the small molecule bound to a label selected from the group consisting of enzymes, fluorophors, chemiluminescers and metallic particles, preferably wherein the small molecule is a fluorescein derivative, the receptor for the small molecule is an antibody, and the label is an enzyme selected from the group consisting of peroxidase, β-galactosidase, urease, alkaline phosphatase and Q-beta-replicase.

The signal generating means can include at least one catalyst, usually an enzyme, and at least one substrate and may include two or more catalysts and a plurality of substrates, and may include a combination of enzymes, where the substrate of one enzyme is the product of the other enzyme. The operation of the signal generating means is to produce a product which provides a detectable signal related to the amount of immunoglobulin in the sample.

A large number of enzymes and coenzyes useful in a signal generating system are indicated in U.S. Patent No. 4,275,149, columns 19 to 23, U.S. Patent No. 4,318,980, columns 10 to 14, and U.S. Patent No. 4,868,104, column 7. A number of enzyme combinations are set forth in U.S. Patent No. 4,275,149, columns 23 to 28, which combinations can find use in the subject invention.

Of particular interest are enzymes which involve the production of hydrogen peroxide and the use of the hydrogen peroxide to oxidize a dye precursor to a dye. Particular combinations include saccharide oxidases, e.g., glucose and galactose oxidase, or heterocyclic oxidases, such as urease and xanthine oxidase, coupled with an enzyme which employs the hydrogen peroxide to oxidize a dye precursor, that is, a peroxidase such as horseradish peroxidase, lactoperoxidase, or microperoxidase. Additional enzyme combinations may be found in the patent references mentioned above. When a single enzyme is used as a label, other enzymes may find use such as hydrolases such as alkaline phosphatase and β-galactosidase. Alternatively, luciferases such as firefly luciferase and bacterial luciferase may be used.

Illustrative coenzymes which find use include NAD[H]; NADP[H], pyridoxal phosphate; FAD[H]; FMN[H], etc., usually coenzymes involving cycling reactions, see particularly U.S. Patent No. 4,318,980.

The product of the enzyme reaction will usually be a dye or fluorescer. A large number of illustrative fluorescers are indicated in U.S. Patent No. 4,275,149, columns 30 and 31.

The signal producing system can include one or more particles, which are insoluble particles of at least about 50 nm and not more than about 50 microns, usually at least about 100 nm and less than about 25 microns, preferably from about 0.2 to 5 microns, diameter. The particle may be organic or inorganic, porous or non-porous, preferably of a density approximating water, generally from about 0.7 to about 1.5 g/ml, and composed of material that can be transparent, partially transparent, or opaque.

The organic particles will normally be comprised of polymers, either addition or condensation polymers, which are readily dispersible in the assay medium. The surface of particles will be adsorptive or functionalizable so as to bind, either directly or indirectly, an oligonucleotide or an sbp member. The nature of particles is described above.

Fluorescers of interest will generally emit light at a wavelength above 350nm, usually above 400nm and preferably above 450nm. Desirably, the fluorescers have a high quantum efficiency, a large Stokes shift and are chemically stable under the conditions of their conjugation and use. The term fluorescer is intended to include substances that emit light upon activation by electromagnetic radiation or chemical activation and includes fluorescent and phosphorescent substances, scintillators, and chemiluminescent substances.

Fluorescers of interest fall into a variety of categories having certain primary functionalities. These primary functionalities include 1- and 2-aminonaphthalene, p,p-diaminostilbenes, pyrenes, quaternary phenanthridine salts, 9-aminoacridines, p,p′-diaminostilbenes immines, anthracenes, oxacarboxyanine, merocyanine, 3-aminoequilenin, perylene, bis-benzoxazole, bis-p-oxazolyl benzene, 1,2-benzophenazine, retinal, bis-3-aminopyridinium salts, hellebrigenin, tetracycline, sterophenol, benzimidazolylphenylamine, 2-oxo-3-chromen, indole, xanthene, 7-hydroxycoumarin, 4,5-benzimidazoles, phenoxazine, salicylate, strophanthidin, porphyrins, triarylmethanes, flavin and rare earth chelates oxides and salts. Exemplary fluorescers are enumerated in U.S. Patent No. 4,318,707, columns 7 and 8.

Additionally, energy absorbent or quenching particles can be employed which are solid insoluble particles of at least about 50 nm in diameter capable of quenching the fluorescence of the fluorescent particle when within the distance resulting from hybridization of a probe with the polynucleotide analyte or from specific binding between members of specific binding pairs. The quenching particle may be the same or different, usually different, from the fluorescent particle. Normally, the quenching particle will provide a substantial quenching at a distance of more than about 50Å, preferably more than about 500Å, more preferably more than about 2000Å, where the distance is measured from the surfaces of the particles.

Many different types of particles may be employed for modulating light emission. Of particular interest are carbon particles, such as charcoal, lamp black, graphite, colloidal carbon and the like. Besides carbon particles metal sols may also find use, particularly of the noble metals, gold, silver, and platinum. Other metal-derived particles may include metal sulfides, such as lead, silver or copper sulfides or metal oxides, such as iron or copper oxide.

An alternative source of light as a detectible signal is a chemiluminescent source. The chemiluminescent source involves a compound which becomes electronically excited by a chemical reaction and may then emit light which serves as the detectible signal or donates energy to a fluorescent acceptor.

A diverse number of families of compounds have been found to provide chemiluminescence under a variety of conditions. One family of compounds is 2,3-dihydro-1,4-phthalazinedione. The most popular compound is luminol, which is the 5-amino analog of the above compound. Other members of the family include the 5-amino-6,7,8-trimethoxy- and the dimethylamine-[ca]benz analog. These compounds can be made to luminesce with alkaline hydrogen peroxide or calcium hypochlorite and base. Another family of compounds is the 2,4,5-triphenylimidazoles, with lophine as the common name for the parent product. Chemiluminescent analogs include para-dimethylamino- and para-methoxy-substituents. Chemiluminescence may also be obtained with oxilates, usually oxalyl, active esters, e.g., p-nitrophenyl and a peroxide, e.g., hydrogen peroxide, under basic conditions. Alternatively, luciferins may be used in conjunction with luciferase or lucigenins.

"Ancillary materials" means various additional materials employed in an assay in accordance with the present invention. For example, buffers will normally be present in the assay medium, as well as stabilizers for the assay medium and the assay components. Frequently, in addition to these additives, additional proteins may be included, such as albumins, or surfactants, particularly non-ionic surfactants, binding enhancers, e.g., polyalkylene glycols, or the like.

"Wholly or partially sequentially" means when the sample and various agents utilized in the present invention are combined other than concomitantly (simultaneously), one or more may be combined with one or more of the remaining agents to form a subcombination. Each subcombination can then be combined and subjected to the present method.

As mentioned above, one aspect of the present invention involves a method for carrying out an immunoassay for a specific immunoglobulin wherein the following components are combined in an aqueous medium: a sample suspected of containing the immunoglobulin, a small molecule bound to a receptor for the immunoglobulin, an antigen capable of binding to the immunoglobulin, a signal generating means bound to a receptor for the antigen where the receptor is capable of binding to a site on the antigen different from the site of binding of the immunoglobulin, and a support to which is bound a receptor for the small molecule. The medium is then separated from the support. The medium or the support is observed for the presence or amount of the signal generating means, the presence or amount thereof being related to the presence or amount of immunoglobulin in the sample. In a preferred practice of the invention, the solid support is a matrix of beads.

The reagents used in the immunoassay of this invention are of particular importance in the design of this invention. As designed, the methodology consists of certain reagents which are generic to most analytes, thereby providing for easier automation and control of stability of reagents in an immunoassay systems. Typically, the present invention uses the following reagents in an assay for an immunoglobulin of interest: (1) a small molecule bound to a receptor for the immunoglobulin, (2) an antigen capable of binding to the immunoglobulin, (3) a signal generating means bound to a receptor for the antigen where the receptor is capable of binding to a site on the antigen different from the site of binding of the immunoglobulin, and (4) a support to which is bound a receptor for the small molecule. If the same small molecule is used in all assays, then the support bound to a receptor is a generic reagent as it can be used regardless of what the analyte is. The signal generating means can consist of a second small molecule bound to a receptor for the antigen along with a labeled receptor for the second small molecule. Once again, if the same second small molecule is used in all assays, then the labeled receptor for the second small molecule is a generic reagent.

In one practice of the invention, a methodology for the detection of an immunoglobulin in a sample is set forth. The methodology of this assay utilizes the following reagents: a sample suspected of containing an immunoglobulin of interest; a small molecule bound to a receptor for the immunoglobulin (Ig), for example, biotin bound to anti-Ig; an antigen capable of binding to Ig; a signal generating means bound to a receptor for the antigen, where the receptor is capable of binding to a site on the antigen different from the binding site of Ig, for example, a label bound to anti-antigen; and a support to which is bound a receptor for the small molecule, for example, avidin. In another embodiment, the signal generating means can be a label bound to a receptor for a small molecule, where the small molecule is bound to anti-antigen, for example, enzyme labeled anti-fluorescein and fluorescein labeled anti-antigen.

The antibodies used as reagents in the present invention can be monoclonal or polyclonal, preferably monoclonal. In such an assay system, the amount of signal generating means that will become bound to the solid support will be directly related to the amount of immunoglobulin present in the sample. In particular, in the presence of immunoglobulin, antigen will be bound at one site by the immunoglobulin. The antibody to which is bound the signal generating means will bind to the antigen at a second, different site. In turn, the anti-Ig, to which is bound a small molecule, binds to the immunoglobulin and the small molecule binds to the receptor for the small molecule, which is bound to the support. This results in the complex of interest binding to the support.

The reagents and sample can be combined simultaneously or wholly or partially sequentially with each other and with the solid support. In one approach, all reagents, the sample and the solid support are mixed together. This is followed by a single incubation, after which unbound reagents are removed by washing. A schematic representation of the complex formed in the immunoassay of the invention as applied to detection of IgM-type immunoglobulins is as follows:

$$\text{Label-Ab}_{Ag}: \text{Ag}: \text{IgM}: \text{Ab}_{IgM}\text{-X}:\text{Y-support}$$

where Label-Ab$_{Ag}$ is an antibody for an antigen (Ag) bound to a label; Ag is an antigen capable of being bound to Ig; Ab$_{IgM}$-X is a small molecule (X) bound to an antibody for Ig; and Y-support is a receptor for the small molecule bound to a support. In a preferred embodiment, the antigen is hepatitis B core antigen or hepatitis A virus antigen. X can be biotin and Y can be avidin.

In one embodiment of the invention, the label, an enzyme for example, can be bound directly to the anti-antigen antibody. The degree of enzymatic activity then correlates to the concentration of IgM in the sample. In a variation on such an assay, a small molecule such as fluorescein, is bound to the anti-antigen antibody. The label is then bound to an anti-fluorescein antibody.

The design of the assay system makes it possible to use reagents which are generic, i.e. can be used in any assay system no matter what the analyte of interest. As is apparent in the above examples, each assay uses the conjugate comprising a receptor bound to a small molecule.

In carrying out the assay there are certain sequences of steps that are more convenient and the choice of the particular sequence to be employed will therefore depend upon the needs of the person performing the assay. In general, the sample, all of the reagents and the support are combined in an aqueous medium, usually without regard to the order of addition. Typically, the sample, reagents and support are combined essentially simultaneously. The aqueous medium containing the sample, reagents and support, is then incubated for a period of up to an hour or more. The amount of signal generating means bound to the support or remaining in the medium may then be measured directly but will usually be measured following separation of the medium from the support.

In the assay of the present invention, any convenient label can be used. The labels will normally be bonded covalently to a receptor for an antigen, usually an antibody. However, this invention also contemplates having a small molecule bound to the receptor for the antigen, and having the label bound covalently to a receptor for the small molecule, usually an antibody. Bonding may be accomplished by chemical reactions wherein the result is replacing a hydrogen atom of the label with a bond to the receptor or may include a linking group between the label and the receptor. The linking group may be of any size but preferably no larger than necessary to permit unfettered binding of a compound complementary to the compound bound to the label and to permit signal production by the label. Generally, the linking group will be a bond or a group of from 1 to 100 atoms, usually from 1 to 15 atoms. The linking group can be introduced into the label or the receptor for attachment. A functionality for attachment such as carboxylic acid, hydroxyl, thio, amino, aldehydic, amido, activated ethylenese such as maleimide, sulfonic acids, and the like can be introduced into the label or the receptor if the functionality is not originally present in the label or receptor. Methods of conjugation are well known in the art. See for example, U.S. Patent No. 3,817,837.

The conjugates of this invention containing a small molecule bound to a receptor, will contain small

7

molecules for which a natural receptor exists or can be prepared. The small molecules will usually be neither extremely hydrophilic nor extremely hydrophobic and will preferably be structurally dissimilar to substances that are likely to be present in the sample. Conjugates of small molecules with receptors will have at least one and frequently 2-20 small molecules in the conjugate which will usually be bound covalently. As described above with reference to the label-receptor bond, bonding of the small molecule to a receptor may be accomplished by chemical reactions which result in replacing a hydrogen atom of the small molecule with a bond to the receptor or may include a linking group between the small molecule and the receptor of any size but preferably no larger than necessary to permit binding to the conjugate of both a receptor for the small molecule and a compound complementary to the receptor in the conjugate.

The support in this invention will normally be particulate such as beads, liposomes, cells, sols, and the like; bibulous materials such as porous membranes, cellulosic paper, glass paper, and nitrocellulose membranes; or non-porous materials such as glass, plastic, metal, ceramic, and the like. The receptor can be directly bonded, either covalently or non-covalently, to the surface of the support in such a manner as to permit ready binding of the conjugate containing a small molecule complementary to the receptor. Bonding of the receptor to the surface will normally be achieved by incubating the surface with the receptor, wherein the surface may previously have been treated with a reagent to enhance binding such as polycations, for example polylysine; carbodiimides; silylating agents; bifunctional cross linking reagents such as carbonyl diimidazole; periodate; and similar activating reagents. Alternatively the receptor can be indirectly bound to the support by first preparing the support with a compound complementary to the receptor such as the ligand for the receptor or an antibody against the receptor already bound to the surface. Incubation of the receptor with such a surface will then cause the receptor to bind non-covalently. When this method of binding is employed wherein a ligand for the receptor is initially present on the surface, the receptor will normally have at least two binding sites for the ligand.

In the invention described herein, when the solid support is a matrix of beads, the beads are usually non-porous, usually glass or latex and normally are 0.2-2.5 mm average in diameter. Most preferably, the beads are 0.5-2 mm average in diameter. The beads are usually approximately spherical and may have a rough or smooth surface.

Because of the high surface area of beads, attention must be paid to the surface properties so that background nonspecific binding remains low. Where avidin is used as the receptor bound to the beads, non-specific binding can be reduced by drying the glass particles in the presence of sucrose after the binding of avidin to the beads. Examples of coatings in addition to sugars, which have been found useful include bovine serum albumin (BSA), poly(vinylalcohol), casein and non-fat milk.

Whatever type of solid support is used it must be treated so as to have a receptor bound to its surface, which receptor will specifically bind to a small molecule. In a preferred practice of the invention, the support will have bound to it a ligand or receptor that will permit the support to be used for a variety of different assays. For example, avidin can be covalently bound to spherical glass beads of 0.5-1.5 mm. A matrix of these beads is mixed in an aqueous medium with biotin-labeled antibodies to an immunoglobulin, a sample containing the immunoglobulin, antigen, and a labeled antibody that will bind to the antigen at a site different from the binding site of the immunoglobulin. Because the beads bind to biotin and biotin can be bound to any antibody, the same beads can be used for most antibody-antigen pairs. After sufficient incubation to permit binding of the labeled antibody to the biotinylated antibody and binding of the latter to the beads, the solution is separated from the beads, for example, by aspiration. Wash solution is then added and liquid is again removed. After repeating the washing cycle, the label is detected and the amount of label is related to the amount of Ig in the sample. For example, if the label is an enzyme, substrate would be added and the amount of enzyme product determined photometrically after a suitable incubation time and compared to the amount of product provided using a sample of known concentration of Ig.

In carrying out the invention, a liquid, usually aqueous, medium will be employed. Other polar solvents may also be employed, usually oxygenated organic solvents from one to six, more usually from one to four, carbon atoms, including alcohols, ethers, and the like. Usually these cosolvents will be present in less than about 40 weight percent, more usually in less than about 20 weight percent. Generally, a pH range of 5 to 10, more usually 6 to 9, will be used. One consideration with respect to the pH of the assay is the maintenance of a significant level of binding while optimizing signal producing proficiency. In some instances, a compromise will be made between these considerations. Various buffers may be used to achieve the desired pH and maintain the pH during the determination. Illustrative buffers include borate, phosphate, carbonate, Tris, barbital, and the like. The particular buffer employed is not critical to this invention; however, in individual separations or individual assays, one buffer may be preferred over another.

Moderate, usually constant, temperatures are normally employed for carrying out the assay. The temperature for the assay, particularly involving an immunoassay, will generally range from about 0-50°C, more usually from about 15-40°C.

8

While the concentrations of the various reagents will generally be determined by the concentration range of the receptors in the liquid medium or of the immunoglobulin in an assay, the final concentration of each of the reagents will normally be determined empirically to optimize the sensitivity and specificity of the separation or of the assay over the range of interest.

In the immunoassay of the invention, the aqueous medium can also contain one or more members of a signal generating means. The concentration of the various members of the signal generating means will vary and be dependent upon the concentration range of interest of the immunoglobulin and the type of measurement or assay involved. As a general point, the concentration of the various members of the signal generating means will be selected to optimize the sensitivity of the assay within the concentration range of interest of the immunoglobulin.

In order to determine the results of the assay, the labeled solid phase reaction product, such as:

$$\text{Label-Ab}_{Ag}: Ag: IgM: Ab_{IgM}\text{-X:Y-support}$$

is preferably separated from the aqueous medium, which may contain free reagents which were present in an excess amount and therefore are unreacted. Since the reaction product is a solid phase insolubilized by the support, it can be readily separated by methods such as sedimentation or centrifugation, or other methods well established in the art.

As a matter of convenience, the reagents for conducting an assay can be provided in a kit in packaged combination in predetermined amounts for use in assaying for an immunoglobulin (Ig). The kit comprises (a) a conjugate of a small molecule and a receptor for Ig, (b) an antigen capable of being bound to Ig, (c) a conjugate of a label and a receptor for the antigen, and (d) a support comprised of a surface bound to a receptor for the small molecule. The kit can also include other reagents for generating a signal in relation to the amount of Ig in the sample, for example, a substrate for the label. Ancillary agents can be included as necessary.

Use of the method of the invention is applicable to any heterogeneous binding assay for an immunoglobulin. Specific assays include for example, assays for IgM antibodies for hepatitis B core antigen and IgM antibodies for hepatitis A virus antigen. In each system, biotinylated antibody that is complementary to the immunoglobulin is used. Receptors other than avidin (which includes streptavidin) may be attached to the beads, such as antibodies, protein A, intrinsic factor, Protein G, C1q, lectins, apoenzymes and the like, whereupon the respective complementary small molecule conjugated to the antibody complementary to the immunoglobulin is then used.

Certain preferred embodiments of th present invention have the following significant advantages over the standard ELISA chemistry: 1) receptor bound supports are generic to all assays; 2) the binding of analyte to antibody in solution phase results in very rapid kinetics compared to those achieved with antibody immobilized on a solid surface; 3) elimination of the need for separate incubations and separate washings provides an efficient assay with a minimum number of manipulative steps. The chemistry to link small molecules such as biotin or fluorescein to antibodies or haptens is simple and efficient. (See, for example, D.M. Boorsma, Immunocytochemistry 2:155 (1983)). The stability of the small molecule conjugates will be as good as the antibodies used in the conjugate.

## Examples

The examples which follow are illustrative and not limiting of the invention. Unless otherwise indicated, reagents were obtained from commercial sources and, where applicable, were used according to manufacturer's directions.

The following abbreviations are used throughout the examples:

| | |
|---|---|
| anti-HAV | anti-hepatitis A virus antibody |
| anti-hIgM | anti-human immunoglobulin antibody |
| anti-HBcAg | anti-hepatitis B core antigen antibody |
| Biotin-LC-NHS | succinimidyl 6-(biotinamido) hexanoate |
| BSA | bovine serum albumin |
| DMF | N,N-dimethyl formamide |
| EDAC | 1-ethyl-(3-dimethylaminopropyl) carbodiimide |
| GB | glass beads |
| HAV | hepatitis A virus |
| HBcAg | hepatitis B core antigen |
| hIgM | human immunoglobulin M |
| HRP | horseradish peroxidase |
| IgG | human immunoglobulin G |
| LC | 3,3'-diamino-N-methyldiproplyamine |
| MES | 2-[N-morpholino]ethane sulfonic acid |

NHS      N-hydroxysuccinimide
PBS      phosphate-buffered saline
TMB      3,3′,5,5′-tetramethylbenzidine

## EXAMPLE 1

Heterogeneous enzyme-based immunoassay for detection of immunoglobulin M for hepatitis B core antigen

### Preparation of Materials

Goat anti-hIgM (affinity purified, m-chain specific) was obtained from Kirkegaard & Perry Laboratories (Gaithersburg, MD). Biotin-anti-hIgM was prepared by reaction of antibody with biotin-LC-NHS. Recombinant HBcAg was obtained from Merck, Sharp and Dohme Research Labs (West Point, PA). Human IgG anti-HBcAg-HRP conjugate is a reagent provided in ETI-CORE-IGMK kits from Sorin Biomedica (Italy). Assay buffer consisted of PBS and 0.1% BSA, pH 7.4. Wash buffer was 10 mM sodium phosphate, pH 7.2. The TMB/urea peroxide substrate was prepared using standard procedures.

### Preparation of GB-avidin

Glass beads of approximately 1 mm in diameter (GlenMills, Inc., Maywood, NJ) were first cleaned by boiling in 5% nitric acid for one hour and then washed with deionized water until the wash was neutral in pH. The beads were dried at room temperature under vacuum.

To 1 kg of the acid-washed beads was added 1 mL of aminopropyltriethoxysilane in 300 mL ethyl acetate. The mixture was then placed on a rotary aspirator, and upon removal of the solvent, the beads were coated with a thin film of the aminosilane reagent. The beads were then transferred to a stainless reactor and heated in an oven at 130°C overnight under nitrogen/argon atmosphere. After cooling, the beads were used directly in the next step.

To 500 g of the aminated beads in a canted tissue culture flask, was added 170 mL 0.1 M of sodium borate (pH 9.0). After 10 minutes, a solution of succinic anhydride (2.0 g in 20 mL DMF) was added by pipette. The flask was capped and shaken manually. All liquid was removed upon the final addition of succinic anhydride solution, and the beads were washed with deionized water 200 mL x 4.

After flushing once with 150 mL 0.1 M MES, pH 5.2, the beads were resuspended in MES to the liquid volume to just cover the beads. One hundred milligrams (100 mg) of EDAC in 2 mL MES was added in one portion and mixed for 5 minutes with manual shaking. Upon removal of the liquid by use of an aspirator, a 20 mL MES solution of avidin (20 mg) and BSA (40 mg) was added in one portion. The beads were mixed manually and more MES buffer was added to just cover the beads. Finally, the culture flask with its contents was placed on an orbital shaker overnight at 4°C.

Further preparation of the beads included washing the beads with 1 N NaCl (200 mL x 4) followed by deionized water (200 mL x 4). Before and after each wash, the liquid is removed entirely. The,beads are then treated with a phosphate-saline buffer (20 mM phosphate, 140 mM NaCl 0.02% NaN3, pH 7.4) containing 0.1% BSA and 2.5% sucrose (150 mL x 3). Excess liquid is removed and the wet beads are transferred to a container in a vacuum dessicator.

Finally, after passage through a number 16 or 20 USA Standard Testing Sieve, the beads were dusted with casein powder to prevent sticking together upon storage.

Binding study with $^3$H-biotin indicated that the beads thus prepared incorporated 2-11 mg active avidin-/glass beads.

### Assay Protocol

The assay protocol was as follows. First, 100 ml of 1:1000 diluted IgM anti-HBcAg positive or negative samples was added to 0.65 g GB-avidin in a test tube, followed by 100 ml of biotin-anti-hIgM (237 ng/assay). Then, 200 ml of the mixture of HBcAg and anti-HBcAg-HRP (containing 100 ng HBcAg/test) was added to each tube. All samples and reagents were diluted in the assay buffer. After a 40 minute incubation at 37°C, the unbound components were removed by washing four times with wash buffer. The bound HRP was measured and stopped with 750 ml of 1 N sulfuric acid. Absorbances were read at 450 nm.

## Results

| Sample | $A_{450}$ |
|--------|-----------|
| Negative | 0.073 |
| Positive | 1.062 |

These results showed clear modulation of the positive sample.

EXAMPLE 2

Heterogeneous enzyme-based immunoassay for detection of immunoglobulin M for hepatitis A virus antigen

Preparation of Materials

The following materials were prepared. Affinity purified biotin labeled goat anti-hIgM (m-chain specific) was obtained from Sigma Diagnostics (St. Louis, MO). Formalin fixed HAV and peroxidase labeled human anti-HAV was provided by Mediagnost (Heidelberg, Germany). GB-avidin was prepared as described in Example 1. The sample dilution buffer and the conjugate diluent was provided in ETI-MAK-IGMK kits from Sorin Biomedica (Italy). Wash buffer was 10 mM sodium phosphate, pH 7.2. The TMB/urea peroxide substrate was prepared using standard procedures.

Preliminary Experiments

Preliminary experiments were performed to determine the appropriate ratio of HAV antigen and peroxidase labeled anti-HAV. Equal amounts of HAV and 1:1500 diluted conjugate provided enough excess of the antigen to bind to captured human anti-HAV.

Proposed Assay Protocol

The proposed assay format would be as follows. First, 200 ml of a mixture of 1:1500 diluted conjugate and HAV antigen would be added to 0.65 g GB-avidin in a test tube. Next, 100 m of 1:1500 diluted anti-HAV IgM positive or negative patient serum would be added to each tube, followed by 100 ml of biotin-anti-hIgM (800 ng/assay). After a 40 minute incubation the unbound components would be removed by washing four times with wash buffer. The bound HRP would be measured by adding 250 ml of TMB/urea peroxide substrate. After a 5 minute incubation at 37°C, the reaction would be stopped with 750 ml of 1 N sulfuric acid. Absorbance at 450 nm would be the measure of bound HRP activity.

The above discussion includes certain theories as to mechanisms involved in the present invention. These theories should not be construed to limit the present invention in any way, since it has been demonstrated that the present invention achieves the results described.

The invention has been described in detail with particular reference to the above embodiments. It will be understood, however, that variations and modifications can be effected within the spirit and scope of the invention.

## Claims

1.  A method for carrying out an immunoassay for a specific immunoglobulin comprising the steps of:
    (a) providing in combination in an aqueous medium (i) a sample suspected of containing an immunoglobulin, (ii) a small molecule bound to a receptor for said immunoglobulin, (iii) an antigen capable of binding to said immunoglobulin, (iv) a signal generating means bound to a receptor for said antigen said receptor being capable of binding to a site on said antigen different from the site of binding of said immunoglobulin, and (v) a support to which is bound a receptor for said small molecule;
    (b) incubating said combination;
    (c) separating said medium and said support; and
    (d) observing said medium or said support for the presence or amount of a signal, the presence or amount thereof being related to the presence or amount of said immunoglobulin in said sample.

2. The method of claim 1 wherein said immunoglobulin is directed against a causative agent of a disease state.

3. The method of claim 1 or claim 2 wherein said immunoglobulin is an IgM antibody.

4. The method of any one of the preceding claims wherein said antigen is a hepatitis B core antigen, or a hepatitis A virus antigen.

5. The method of any one of the preceding claims wherein said signal generating means is a label selected from the group consisting of enzymes, fluorophors, chemiluminescers and metallic particles, preferably wherein said label is an enzyme selected from the group consisting of peroxidase, β-galactosidase, urease, alkaline phosphatase and Q-beta-replicase.

6. The method of any one of claims 1 to 4 wherein said signal generating means comprises a second small molecule bound to said receptor for said antigen and a receptor for said small molecule bound to a label selected from the group consisting of enzymes, fluorophors chemiluminescers and metallic particles, preferably wherein said second small molecule is a fluorescein derivative, said receptor for the small molecule is an antibody, and said label is an enzyme selected from the group consisting of peroxidase, β-galactosidase, urease and alkaline phosphatase.

7. The method of any one of the preceding claims wherein said receptor for said immunoglobulin is an antibody.

8. The method of any one of the preceding claims 5 wherein said receptor for said antigen is an antibody.

9. The method of any of the preceding claims wherein said small molecule is biotin, and wherein said receptor for said small molecule is selected from the group consisting of avidin and streptavidin.

10. The method of any one of the preceding claims wherein said support is glass beads.

11. A method for carrying out an immunoassay for an IgM-type immunoglobulin comprising the steps of:
(a) providing in an assay medium (i) a sample suspected of containing IgM, (ii) a biotinylated antibody capable of binding said IgM, (iii) an antigen:antibody complex wherein said antigen is capable of binding to said IgM and said antibody is bound to a signal generating means and (iv) a receptor for biotin bound to a support;
(b) incubating said medium;
(c) separating said medium and said support; and
(d) observing said medium or support for the presence or amount of a signal, the presence or amount thereof being related to the presence or amount of IgM in said sample.

12. An assay for detecting an IgM-type immunoglobulin suspected of being present in a sample comprising the step of forming in direct relation to the amount of said IgM in said sample a complex
$$\text{Label-Ab}_{Ag}: \text{Ag}: \text{IgM}: \text{Ab}_{IgM}\text{-X}:\text{Y-support}$$
wherein Label-Ab$_{Ag}$ is an antibody for an antigen (Ag) bound to a label; Ag is an antigen capable of being bound to IgM and is selected from the group consisting of hepatitis A virus antigen and hepatitis B core antigen; Ab$_{IgM}$-X is a small molecule (X) bound to an antibody for said IgM; and Y-support is a receptor for said small molecule bound to a support.

13. A composition of matter consisting of a conjugate of a biotinylated antibody bound to an immunoglobulin, an antigen bound to said immunoglobulin and a labeled antibody bound to said antigen.

14. The composition of claim 13 wherein said biotinylated antibody is further bound to a support having a receptor for biotin, said receptor being selected from the group consisting of avidin and streptavidin.

15. A composition of matter:
$$\text{Label-Ab}_{Ag}: \text{Ag}: \text{IgM}: \text{Ab}_{IgM}\text{-X}:\text{Y-support}$$
wherein Label-Ab$_{Ag}$ is an antibody for an antigen (Ag) bound to a label; IgM is an IgM-type immunoglobulin; Ag is an antigen capable of being bound to IgM and is selected from the group consisting of hepatitis A virus antigen and hepatitis B core antigen; Ab$_{IgM}$-X is a small molecule (X) bound to an antibody for said IgM; and Y-support is a receptor for said small molecule bound to a support.

16. A kit for carrying out an immunoassay for an immunoglobulin comprising in packaged form: (a) a conjugate of a small molecule and a receptor for said immunoglobulin; (b) an antigen capable of being bound to said immunoglobulin; (c) a conjugate of a label and a receptor for said antigen; and (d) a support comprised of a surface bound to a receptor for said small molecule.